# EUROPEAN PATENT SPECIFICATION

(11) **EP 1 278 063 B1**
(45) Date of publication and mention of the grant of the patent: **19.04.2006**
(21) Application number: 01202713.2
(22) Date of filing: 16.07.2001
(51) Int. Cl.: G01N 33/543

(54) **Imobilization of biomolecules**
Immobilisierung von Oligonukleotiden durch kovalente Bindung
Immobilisation d'oligonucléotides via liaison covalente

(43) Date of publication of application: 22.01.2003
(73) Proprietor: DR. Chip Biotechnology Inc., Hsinchu, Taiwan 300, R.O.C. (TW)
(72) Inventor: Lee, Kan-Hung, 2F, No. 3-2 Industry E. 9th Road, Hsinchu, Taiwan 300 (TW); Chen, Kang-Jehng, 2F, No. 3-2 Industry E. 9th Road, Hsinchu, Taiwan 300 (TW); Hsiao, Hsing, 2F, No. 3-2 Industry E. 9th Road, Hsinchu, Taiwan 300 (TW); Wang, Shin-Hwan, 2F, No. 3-2 Industry E. 9th Road, Hsinchu, Taiwan 300 (TW)
(74) Representative: Lunt, Mark George Francis

(56) References cited:
- EP-A- 0 390 500
- EP-A- 0 403 700
- EP-A- 1 035 218
- WO-A-01/16372
- WO-A-01/40310
- BASU S ET AL: "Solid Phase Synthesis of a d-Peptide-Phosphorothioate Oligodeoxynucleotide Conjugate from Two Arms of a Polyethylene Glycol-Polystyrene Support" TETRAHEDRON LETTERS, ELSEVIER SCIENCE PUBLISHERS, AMSTERDAM, NL, vol. 36, no. 28, 10 July 1995 (1995-07-10), pages 4943-4946, XP004027705 ISSN: 0040-4039
- DATABASE WPI Section Ch, Week 199149 Derwent Publications Ltd., London, GB; Class A14, AN 1991-356630 XP002195605 & JP 03 237147 A (MITSUBISHI KASEI VINYL KK), 23 October 1991 (1991-10-23)
- LEMIEUX ET AL: "Chemoselective ligation reactions with proteins, oligosaccharides and cells" TIBTECH, vol. 16, 1998, pages 506-513,

## Description

Diagnostic assays and other chemical processes often require attaching a molecule to a solid support. For example, a protein is commonly attached to a solid support for an immune assay, and an oligonucleotide to a solid support for a hybridization-based assay. The attachment can be achieved in a number of different ways, including covalent bonding and non-covalent interaction. Typically, covalent attachment is more robust. See, for example, Lamture *et al.* (1994) *Oligonucleotide Research* 22: 2121-2125; Beattie *et al.* (1995) *Mol. Biotechnol.* 4: 213-225; Joos *et al.* (1997) *Anal. Biochem* 247: 96-101; Rogers *et al.* (1999) *Anal. Biochem.* 266: 23-30; and Chrisey *et al.* (1996) *Oligonucleotide Research* 24: 3031-3039.

A number of protocols have been developed to covalently attach an oligonucleotide to a support surface. One example achieves this by, e.g., synthesizing an oligonucleotide directly on a support surface using stepwise photolithographic reactions. For example, see U.S. Patent Nos. 5,424,186; 5,510,270; and 5,744,305. Alternatively, a nucleic acid, such as a cloned cDNA, a PCR product, or a synthetic oligonucleotide, can be deposited onto a surface of a solid support, e.g., a microscopic glass slide, in the form of an array. Usually, the surface is modified in order to covalently attach a nucleic acid.

EP0390500 discloses immobilizing proteins (e.g., human IgG) on a polystyrene surface modified with a third molecule. i.e., a N-hydroxymethyl acetamide compound

EP0403700 discloses reacting ligands (e.g., nucleic acids or proteins) with silica gel modified with third and fourth molecules.

The silica gel is first modified with polyethyleneiminopropyl trimethoxysilane and then with a compound having a reactive moiety that is capable of forming covalent linkages with ligands (e.g., glutaraldehyde). EP1035218 discloses immobilizing an oligonucleotide on a surface modified with a third molecule. i.e., a polymer containing functional groups. Basu and Wickstrom, Tetrahedron Letters, 3682,4943 - 4946, 1995, disclose synthesizing a DNA-peptide conjugate on an amino-HLP support modified with a third molecule, 1-(Fmoc-amino)-5-(hydroxymethyl)-6-(DMT-O)-hexane,

In one aspect, this invention relates to a biomolecule-bound substrate that includes (1) a solid support made of polymeric molecules, each of which contains a reacting group; and (2) a plurality of biomolecules, each of which contains another reacting group. One of the two reacting groups is a leaving group and the other is a substitute group, and the biomolecules are covalently bonded to the solid support via a chemical ligation reaction between the two reacting groups. In other words, after the chemical ligation reaction, the leaving group departs from the biomolecules or the solid support, and the substitute group bridges the biomolecules and the solid support via a covalent bond. Each of the two just-described reacting groups refers to either its pre- or post- reaction state, depending on whether it participates in the reaction. Indeed, in a biomolecule-bound substrate of this invention, not all reacting groups on the substrate participate in the ligation reaction.

A leaving group is the group that departs from a molecule during a chemical ligation reaction. It can be, for example, halogen. A substitute group can be either a nucleophilic group or an electrophilic group. A nucleophilic group is a chemical species having unshared pair electrons (e.g., any Lewis base), and can be neutral or have a negative charge. Examples of the nucleophilic group include an oxygen-containing group (e.g., hydroxyl, alkoxy, or acyloxy), a sulfur-containing group (e.g., mercapto, alkylthio, sulfonate, or phosphorothioate), a nitrogen-containing group (e.g., amino, alkylamino, acylamino, nitro, azido, or isocyanato), and halogen. An electrophilic group is a chemical species having a vacant orbital for electrons to occupy, and can be neutral or have a positive charge. An example of the electrophilic group is an organometal.

A solid support used to practice this invention is made of at least one type of polymeric molecules evenly distributed throughout the solid support, each of which contains a leaving group or a substitute group. A solid support can be flexible and capable of being bent, folded, or otherwise manipulated without breakage. It can also be rigid and takes on a desirable configuration, such as film, sheet, tube, disc, or sphere. A porous solid support, such as gel, can also be used. Any of the just-described solid support can be used alone, or in combination with any other support (e.g., glass) well known in the art. Examples of a solid support for use in this invention include, but are not limited to, polyvinyl chloride resin (PVC), urea-formaldehyde resin, and acrylic. When a solid support is PVC resin, the chloride group in the PVC is a leaving group. A biomolecule containing a substitute group can react with the chloride group, resulting in covalently bonding of the biomolecule to the PVC resin. When a solid support is urea-formaldehyde resin, the amino group in urea formaldehyde is a substitute group. A biomolecule containing a leaving group can react with the amino group, resulting in covalently bonding of the biomolecule to the urea-formaldehyde resin.

A biomolecule to be attached to the just-described solid support can be a biopolymer (e.g., an oligonucleotide, a peptide, a polysaccharide, or a glycoprotein) or a biomonomer (e.g., a nucleoside, an amino acid, or a monosaccharide), any of which can be a naturally occurring molecule or a synthetic analog. The term "oligonucleotide" used herein refers a synthetic DNA, a synthetic RNA, a cDNA, an mRNA, or a peptide nucleic acid. The biomolecule contains a leaving group or a substitute group, which, if not present naturally, must be introduced by chemical or biochemical methods well known in the art. A leaving group or a substitute group can locate at any suitable position of the biomolecule.

In another aspect, this invention relates to a method for preparing the afore-described biomolecule-bound substrate. The method includes (1) providing a solid support made of polymeric molecules, each of which contains a leaving group (or a substitute group), said group being present without modification of the surface of the solid support; and a plurality of biomolecules, each of which contains a substitute group (or a leaving group); and (2) bonding the biomolecules to the solid support via a chemical ligation reaction between the leaving and substitute groups to form a biomolecule-bound substrate.

One advantage of this invention is that the surface of a solid support need not be modified in order to covalently attach a biomolecule. Other advantages, features, and objects of the invention will be apparent from the description and from the claims.

The details of one or more embodiments of the invention are set forth in the description below.

A biomolecule-bound substrate of this invention can be prepared by covalently bonding a biomolecule containing a substitute group to a solid support containing a leaving group. For example, one can deposit a phosphorothioate-containing oligonucleotide onto PVC resin. The phosphorothioate group, a substitute group, reacts with the chloride group, a leaving group, in the PVC to form an oligonucleotide-bound PVC resin. Shown below is a scheme that depicts this chemical ligation reaction.

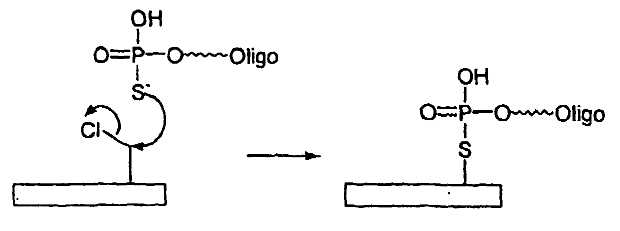

A biomolecule-bound substrate of this invention also can be prepared by covalently bonding a biomolecule containing a leaving group to a solid support containing a substitute group. For example, one can deposit an iodothymidine-containing oligonucleotide onto urea-formaldehyde resin. The amino group, a substitute group, in the urea-formaldehyde resin reacts with the iodo group, a leaving group, on the oligonucleotide to produce oligonucleotide-bound urea-formaldehyde resin. Shown below is a scheme that depicts this chemical ligation reaction.

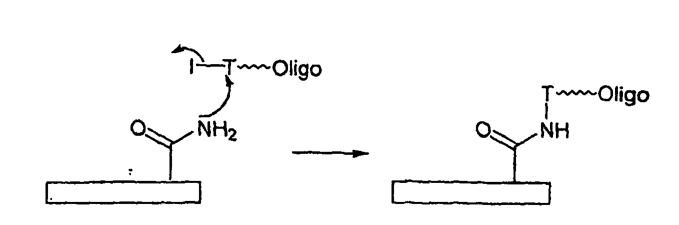

A biomolecule has a reacting group (i.e., a leaving group or a substitute group), which can locate at any suitable position. For example, an oligonucleotide has a reacting group at its the 3' or the 5' terminus, or at a non-terminal position. It can be immobilized onto a solid support and hybridize with its pair member. When the reacting group is at a non-terminal position, the oligonucleotide may be capable of forming a "hairpin" structure on the solid support to improve hybridization efficacy.

A biomolecule that contain a reacting group can be prepared using any convenient methodology. For example, wherein the biomolecule is an oligonucleotide, a number of protocols exist for introducing an oligonucleotide with a reacting group, if not present naturally. For instance, an oligonucleotide can be chemically synthesized on a DNA/RNA synthesizer using non-modified phosphoramidites, and a reacting group can be enzymatically added to one of the termini of the oligonucleotide. Alternatively, a modified phosphoramidite, with a reacting group, can be incorporated into a suitable position of an oligonucleotide using a DNA/RNA synthesizer. As another example, where the biomolecule is a peptide, it can be prepared chemically (e.g., on a peptide synthesizer) or biologically (e.g., expressed from a host cell). A functional group such as carboxy, hydroxy, phenoxy, amino, guanidino, or mercapto is present in peptides, and can serve as a reacting group. If an additional reacting group is needed, it can be introduced by, for example, incorporation of an amino acid analog.

Synthesis of a backbone-modified oligonucleotide, such as a phosphorothioate-containing oligonucleotide, is described in, for example, Krieg *et al.* (1995) *Nature* 374: 546-549; Weiner *et al.* (1997) *Proc. Natl. Acad Sci. USA* 94: 10833-10837; and Boggs *et al.* (1997) *Antisense Nucleic Acid Drug Dev* 7(5): 461-471. Synthesis of a base-modified oligonucleotide, as well as other backbone-modified oligonucleotides (e.g., containing phosphorodithioate or aminoalkylphosphotriester), is described in, for example, U.S. Patent 6,232,296.

A biomolecule can be bound to the solid support randomly, or in an order. The method of this invention can introduce a biomolecule, such as an oligonucleotide, a peptide, a polysaccharide, a nucleoside, an amino acid, or a monosaccharide, on a solid support in the form of an array, i.e., an orderly arrangement such as a matrix of rows and columns. An individual array can contain a number of unique attached biomolecules. The array may contain a plurality of addresses (each address being a unique attached biomolecule), and one or more unique attached molecules. Each addressable site can be directly adjacent to at least one other site, or can be separated from each other site, e.g., by a ridge, etch or surface lacking attached biomolecules. The array can have a plurality of addresses on the solid support. The density of the addresses is selected to provide for adequate resolution for detection, and can be at least 10, 10³, 10⁵, 10⁷ or 10⁹ addresses/cm², or no more than 10, 10³, 10⁵, 10⁷ or 10⁹ addresses/cm². The center to center distance between addresses can be 1 cm, 10 mm, 10 nm, 0. 1 nm or less, or ranges between. The longest diameter of each address can be 1 cm, 10 mm, 10 nm, 0.1 nm or less, or ranges between. Each address can contain 10 mg, 100 ng, 100 pg, 0.1 pg or less of the biomolecule, or ranges between. Alternatively, each address contains 100, 10⁴, 10⁶, 10⁸ or more biomolecules, or ranges between. The addresses can be distributed, on the substrate in one dimension, in two dimensions, or in three dimensions.

A biomolecule-bound substrate of this invention can be used in variety applications, where such applications are generally analytical in which the presence of a particular analyte in a given sample is detected at least qualitatively, if not quantitatively. More specifically, the sample suspected of containing the analyte of interest is contacted with the biomolecules on the solid support under conditions sufficient for the analyte to interact with its respective pair member that is present on the solid support. If the analyte of interest is present in the sample, it can form a complex with its pair member. The presence of the complex can be detected by, e.g., use of a signal production system such as an isotopic or fluorescent label present on the analyte.

An example of a biomolecule-bound substrate is an oligonucleotide array, in which a hybridization assay can be employed. The hybridization assay can be a gene discovery assay, a differential gene expression analysis assay, a sequencing assay, or an analysis of genomic polymorphism. For example, an oligonucleotide array can be used to produce gene expression profiles after polymerase mediated primer extension reactions. See, e.g., U.S. Patent No. 5,262,311; Liang, P & Pardee, A.B. (1992) *Science* 257; 967-971; Liang, P & Pardee, A.B. eds. (1997) *Methods in Molecular Biology:* Differential Display Methods and Protocols, Vol 85.). Such an array can be used, for example, to identify genes associated with diseases or screen compounds for drug discovery in a high throughput format. In particular, a high-density array has been proven to monitor gene expression, map genomic library clones, and resequence genes to screen for mutations and polymorphisms. For example, see, Ramsay (1998) *Nature Biotechnology* 16: 40-44; Bains and Smith (1988) *J. Theor. Biol.* 135: 303-307; Drmanac *et al.* (1989) *Genomics* 4: 114-128; and Shena *et al.* (1995) *Science* 270: 467-470.

The specific examples below are to be construed as merely illustrative, and not limitative of the remainder of the disclosure in any way whatsoever. Without further elaboration, it is believed that one skilled in the art can, based on the description herein, utilize the present invention to its fullest extent.

### Example 1: Preparation of an oligonucleotide-bound plastic substrate

A 10µM (TM) 3'-phosphorothioate and 5'-biotin modified oligonucleotide solution was prepared. 0.5µL (TL) of the solution was spotted onto a plastic substrate made of PVC resin. The plastic chip was incubated at 32°C overnight, washed with deionized distilled water (DDW), reacted with streptavidin-alkaline phosphatase, and treated with a buffer containing nitroblue tetrazolium (NBT)/5-bromo-4-chloro-3-indolylphosphate (BCIP). The results indicate that oligonucleotide was covalently bound to the plastic substrate.

### Example 2: Preparation of an oligonucleotide-bound substrate.

A 10µM (TM) 3'-amino and 5'-biotin modified oligonucleotide solution was prepared. 0.5 µL (TL) of the solution was spotted onto a plastic chip made of PVC resin. The plastic substrate was incubated at 32°C overnight, washed with DDW, reacted with streptavidin-alkaline phosphatase, and treated with a buffer containing NBT/BCIP. The results indicate that oligonucleotide was covalently bound to the plastic substrate.

### Example 3: Hybridization of a bound oligonucleotide on a plastic substrate.

A 10µM (TM) 3'-phosphorothioate modified oligonucleotide solution was prepared. 0.5 TL of the solution was spotted onto a plastic chip made of PVC resin. The plastic substrate was incubated at 32°C overnight, and washed with DDW. A biotin-labeled PCR product, containing a sequence complementary to the 3'-phosphorothioate modified oligonucleotide, was applied to the plastic substrate. After incubation, the chip was washed, reacted with streptavidin-alkline phosphatase, and treated with a detection buffer containing NBT/BCIP. The results showed that hybridization of the PCR product to the oligonucleotide on the substrate was efficient.

## Claims

1. A biomolecule-bound substrate comprising:
a solid support made of polymeric molecules, each of which contains a first reacting group, said first reacting group being present without modification of the surface of the solid support; and
a plurality of biomolecules, each of which contains a second reacting group;
wherein one of the two reacting groups is a substitute group and the other is a leaving group; and the biomolecules are covalently bonded to the solid support via a chemical ligation reaction between the two reacting groups.

2. The biomolecule-bound substrate of claim 1, wherein the first reacting group is a leaving group and the second reacting group is a nucleophilic group.

3. The biomolecule-bound substrate of claim 2, wherein the first reacting group is halogen, amino, or mercapto.

4. The biomolecule-bound substrate of claim 3, wherein the solid support is polyvinyl chloride and the first reacting group is chloride.

5. The biomolecule-bound substrate of claim 2, wherein the second reacting group is phosphorothioate or an amino.

6. The biomolecule-bound substrate of claim 5, wherein the first reacting group is halogen, amino, or mercapto.

7. The biomolecule-bound substrate of claim 6, wherein the solid support is polyvinyl chloride and the first reacting group is chloride.

8. The biomolecule-bound substrate of claim 2, wherein the biomolecule is a nucleotide or an oligonucleotide.

9. The biomolecule-bound substrate of claim 8, wherein the second reacting group is phosphorothioate or an amino.

10. The biomolecule-bound substrate of claim 9, wherein the first reacting group is halogen, amino, or mercapto.

11. The biomolecule-bound substrate of claim 10, wherein the solid support is polyvinyl chloride and the first reacting group is chloride.

12. The biomolecule-bound substrate of claim 2, wherein the biomolecule is an amino acid or a peptide.

13. The biomolecule-bound substrate of claim 1, wherein the first reacting group is a leaving group and the second reacting group is an electrophilic group.

14. The biomolecule-bound substrate of claim 1, wherein the first reacting group is a nucleophilic group and the second reacting group is a leaving group.

15. The biomolecule-bound substrate of claim 14, wherein the first reacting group is halogen, amino, or mercapto.

16. The biomolecule-bound substrate of claim 15, wherein the solid support is urea-formaldehyde resin and the first reacting group is amino.

17. The biomolecule-bound substrate of claim 14, wherein the second reacting group is halogen.

18. The biomolecule-bound substrate of claim 17, wherein the first reacting group is halogen, amino, or mercapto.

19. The biomolecule-bound substrate of claim 18, wherein the solid support is urea-formaldehyde resin and the first reacting group is amino.

20. The biomolecule-bound substrate of claim 14, wherein the biomolecule is a nucleotide or an oligonucleotide.

21. The biomolecule-bound substrate of claim 20, wherein the second reacting group is halogen.

22. The biomolecule-bound substrate of claim 21, wherein the first reacting group is halogen, amino, or mercapto.

23. The biomolecule-bound substrate of claim 22, wherein the solid support is urea-formaldehyde resin and the first reacting group is amino.

24. The biomolecule-bound substrate of claim 14, wherein the biomolecule is an amino acid or a peptide.

25. The biomolecule-bound substrate of claim 1, wherein the first reacting group is an electrophilic group and the second reacting group is a leaving group.

26. A method for preparing a biomolecule-bound substrate, comprising:
providing a solid support made of polymeric molecules, each of which contains a first reacting group, said first reacting group being present without modification of the surface of the solid support; and a plurality of biomolecules, each of which contains a second reacting group; wherein one of the two reacting groups is a substitute group and the other is a leaving group; and
bonding the biomolecules to the solid support via a chemical ligation reaction between the two reacting groups to form the biomolecule-bound substrate.

27. The method of claim 26, wherein the first reacting group is a leaving group and the second reacting group is a nucleophilic group.

28. The method of claim 27, wherein the solid support is polyvinyl chloride and the first reacting group is chloride.

29. The method of claim 27, wherein the second reacting group is phosphorothioate or an amino.

30. The method of claim 29, wherein the solid support is polyvinyl chloride and the first reacting group is chloride.

31. The method of claim 27, wherein the biomolecule is a nucleotide or an oligonucleotide.

32. The method of claim 27, wherein the biomolecule is an amino acid or a peptide.

33. The method of claim 26, wherein the first reacting group is a leaving group and the second reacting group is an electrophilic group.

34. The method of claim 26, wherein the first reacting group is a nucleophilic group and the second reacting group is a leaving group.

35. The method of claim 34, wherein the biomolecule is a nucleotide or an oligonucleotide.

36. The method of claim 34, wherein the biomolecule is an amino acid or a peptide.

37. The method of claim 26, wherein the first reacting group is an electrophilic group and the second reacting group is a leaving group.

## Revendications

1. Substrat à biomolécules liées comprenant :
- un support solide fait de molécules polymériques, dont chacune contient un premier groupe réactif, ledit premier groupe réactif étant présent sans modification de la surface du support solide ; et
- une pluralité de biomolécules, dont chacune contient un second groupe réactif ;
dans lequel l'un des deux groupes réactifs est un groupe de substitution et l'autre est un groupe partant ; et les biomolécules sont liées de façon covalente au support solide par l'intermédiaire d'une réaction de liaison chimique entre les deux groupes réactifs.

2. Substrat à biomolécules liées selon la revendication 1, dans lequel le premier groupe réactif est un groupe partant et le second groupe réactif est un groupe nucléophile.

3. Substrat à biomolécules liées selon la revendication 2, dans lequel le premier groupe réactif est halogène, amino ou mercapto.

4. Substrat à biomolécules liées selon la revendication 3, dans lequel le support solide est du poly(chlorure de vinyle) et le premier groupe réactif est chlorure.

5. Substrat à biomolécules liées selon la revendication 2, dans lequel le second groupe réactif est phosphorothioate ou amino.

6. Substrat à biomolécules liées selon la revendication 5, dans lequel le premier groupe réactif est halogène, amino ou mercapto.

7. Substrat à biomolécules liées selon la revendication 6, dans lequel le support solide est du poly(chlorure de vinyle) et le premier groupe réactif est chlorure.

8. Substrat à biomolécules liées selon la revendication 2, dans lequel la biomolécule est un nucléotide ou un oligonucléotide.

9. Substrat à biomolécules liées selon la revendication 8, dans lequel le second groupe réactif est phosphorothioate ou amino.

10. Substrat à biomolécules liées selon la revendication 9, dans lequel le premier groupe réactif est halogène, amino ou mercapto.

11. Substrat à biomolécules liées selon la revendication 10, dans lequel le support solide est du poly(chlorure de vinyle) et le premier groupe réactif est chlorure.

12. Substrat à biomolécules liées selon la revendication 2, dans lequel la biomolécule est un acide aminé ou un peptide.

13. Substrat à biomolécules liées selon la revendication 1, dans lequel le premier groupe réactif est un groupe partant et le second groupe réactif est un groupe électrophile.

14. Substrat à biomolécules liées selon la revendication 1, dans lequel le premier groupe réactif est un groupe nucléophile et le second groupe réactif est un groupe partant.

15. Substrat à biomolécules liées selon la revendication 14, dans lequel le premier groupe réactif est halogène, amino ou mercapto.

16. Substrat à biomolécules liées selon la revendication 15, dans lequel le support solide est une résine urée-formaldéhyde et le premier groupe réactif est amino.

17. Substrat à biomolécules liées selon la revendication 14, dans lequel le second groupe réactif est halogène.

18. Substrat à biomolécules liées selon la revendication 17, dans lequel le premier groupe réactif est halogène, amino ou mercapto.

19. Substrat à biomolécules liées selon la revendication 18, dans lequel le support solide est une résine urée-formaldéhyde et le premier groupe réactif est amino.

20. Substrat à biomolécules liées selon la revendication 14, dans lequel la biomolécule est un nucléotide ou un oligonucléotide.

21. Substrat à biomolécules liées selon la revendication 20, dans lequel le second groupe réactif est halogène.

22. Substrat à biomolécules liées selon la revendication 21, dans lequel le premier groupe réactif est halogène, amino ou mercapto.

23. Substrat à biomolécules liées selon la revendication 22, dans lequel le support solide est une résine urée-formaldéhyde et le premier groupe réactif est amino.

24. Substrat à biomolécules liées selon la revendication 14, dans lequel la biomolécule est un acide aminé ou un peptide.

25. Substrat à biomolécules liées selon la revendication 1, dans lequel le premier groupe réactif est un groupe électrophile et le second groupe réactif est un groupe partant.

26. Procédé de préparation d'un substrat à biomolécules liées, comprenant :
- prendre un support solide fait de molécules polymériques, dont chacune contient un premier groupe réactif, ledit premier groupe réactif étant présent sans modification de la surface du support solide ; et une pluralité de biomolécules, dont chacune contient un second groupe réactif ; dans lequel l'un des deux groupes réactifs est un groupe de substitution et l'autre est un groupe partant ; et
- lier les biomolécules au support solide par l'intermédiaire d'une réaction de liaison chimique entre les deux groupes réactifs afin de former le substrat à biomolécules liées.

27. Procédé selon la revendication 26, dans lequel le premier groupe réactif est un groupe partant et le second groupe réactif est un groupe nucléophile.

28. Procédé selon la revendication 27, dans lequel le support solide est du poly(chlorure de vinyle) et le premier groupe réactif est chlorure.

29. Procédé selon la revendication 27, dans lequel le second groupe réactif est phosphorothioate ou amino.

30. Procédé selon la revendication 29, dans lequel le support solide est du poly(chlorure de vinyle) et le premier groupe réactif est chlorure.

31. Procédé selon la revendication 27, dans lequel la biomolécule est un nucléotide ou un oligonucléotide.

32. Procédé selon la revendication 27, dans lequel la biomolécule est un acide aminé ou un peptide.

33. Procédé selon la revendication 26, dans lequel le premier groupe réactif est un groupe partant et le second groupe réactif est un groupe électrophile.

34. Procédé selon la revendication 26, dans lequel le premier groupe réactif est un groupe nucléophile et le second groupe réactif est un groupe partant.

35. Procédé selon la revendication 34, dans lequel la biomolécule est un nucléotide ou un oligonucléotide.

36. Procédé selon la revendication 34, dans lequel la biomolécule est un acide aminé ou un peptide.

37. Procédé selon la revendication 26, dans lequel le premier groupe réactif est un groupe électrophile et le second groupe réactif est un groupe partant.

## Patentansprüche

1. Substrat mit daran gebundenem Biomolekül, umfassend:
einen festen Träger bestehend aus polymeren Molekülen, von denen jedes eine erste reaktive Gruppe enthält, wobei die erste reaktive Gruppe ohne Änderung der Oberfläche des festen Trägers vorliegt; und
eine Mehrzahl an Biomolekülen, von denen jedes eine zweite reaktive Gruppe enthält;
wobei eine der zwei reaktiven Gruppen einen Substituenten und die andere eine Abgangsgruppe darstellt; und die Biomoleküle mittels einer chemischen Bindungsreaktion zwischen den zwei reaktiven Gruppen kovalent an den festen Träger gebunden sind.

2. Das Substrat mit daran gebundenem Biomolekül gemäß Anspruch 1, bei dem die erste reaktive Gruppe eine Abgangsgruppe und die zweite reaktive Gruppe eine nukleophile Gruppe ist.

3. Das Substrat mit daran gebundenem Biomolekül gemäß Anspruch 2, bei dem die erste reaktive Gruppe ein Halogen, eine Amino- oder Mercapto-Gruppe darstellt.

4. Substrat mit daran gebundenem Biomolekül gemäß Anspruch 3, bei dem der feste Träger Polyvinylchlorid und die erste reaktive Gruppe ein Chlorid ist.

5. Substrat mit daran gebundenem Biomolekül gemäß Anspruch 2, bei dem die zweite reaktive Gruppe eine Phosphorthioat- oder eine Aminogruppe ist.

6. Substrat mit daran gebundenem Biomolekül gemäß Anspruch 5, bei dem die erste reaktive Gruppe Halogen, eine Amino- oder eine Mercapto-Gruppe ist.

7. Substrat mit daran gebundenem Biomolekül gemäß Anspruch 6, bei dem der feste Träger Polyvinylchlorid und die erste reaktive Gruppe Chlorid ist.

8. Substrat mit daran gebundenem Biomolekül gemäß Anspruch 2, bei dem das Biomolekül ein Nukleotid oder ein Oligonukleotid ist.

9. Substrat mit daran gebundenem Biomolekül gemäß Anspruch 8, bei dem die zweite reaktive Gruppe eine Phosphorthioat- oder eine Amino-Gruppe ist.

10. Substrat mit daran gebundenem Biomolekül gemäß Anspruch 9, bei dem die erste reaktive Gruppe ein Halogen, eine Amino- oder Mercapto-Gruppe ist.

11. Substrat mit daran gebundenem Biomolekül gemäß Anspruch 10, bei dem der feste Träger Polyvinylchlorid und die erste reaktive Gruppe Chlorid ist.

12. Substrat mit daran gebundenem Biomolekül gemäß Anspruch 2, bei dem das Biomolekül eine Aminosäure oder ein Peptid ist.

13. Substrat mit daran gebundenem Biomolekül gemäß Anspruch 1, bei dem die erste reaktive Gruppe eine Abgangsgruppe und die zweite reaktive Gruppe eine elektrophile Gruppe ist.

14. Substrat mit daran gebundenem Biomolekül gemäß Anspruch 1, bei dem die erste reaktive Gruppe eine nukleophile Gruppe und die zweite reaktive Gruppe eine Abgangsgruppe ist.

15. Substrat mit daran gebundenem Biomolekül gemäß Anspruch 14, bei dem die erste reaktive Gruppe ein Halogen, eine Amino- oder eine Mercapto-Gruppe ist.

16. Substrat mit daran gebundenem Biomolekül gemäß Anspruch 15, bei dem der Träger ein Harnstoff-Formaldehydharz und die erste reaktive Gruppe eine Amino-Gruppe ist.

17. Substrat mit daran gebundenem Biomolekül gemäß Anspruch 14, bei dem die zweite reaktive Gruppe ein Halogen ist.

18. Substrat mit daran gebundenem Biomolekül gemäß Anspruch 17, bei dem die erste reaktive Gruppe Halogen, eine Amino- oder eine Mercapto-Gruppe ist.

19. Substrat mit daran gebundenem Biomolekül gemäß Anspruch 18, bei dem der feste Träger ein Harnstoff-Formaldehydharz und die erste reaktive Gruppe eine Amino-Gruppe ist.

20. Substrat mit daran gebundenem Biomolekül gemäß Anspruch 14, bei dem das Biomolekül ein Nukleotid oder ein Oligonukleotid ist.

21. Substrat mit daran gebundenem Biomolekül gemäß Anspruch 20, bei dem die zweite reaktive Gruppe ein Halogen ist.

22. Substrat mit daran gebundenem Biomolekül gemäß Anspruch 21, bei dem die erste reaktive Gruppe ein Halogen, eine Amino- oder eine Mercapto-Gruppe ist.

23. Substrat mit daran gebundenem Biomolekül gemäß Anspruch 22, bei dem der feste Träger ein Harnstoff-Formaldehydharz und die erste reaktive Gruppe eine Amino-Gruppe ist.

24. Substrat mit daran gebundenem Biomolekül gemäß Anspruch 14, bei dem das Biomolekül eine Aminosäure oder ein Peptid ist.

25. Substrat mit daran gebundenem Biomolekül gemäß Anspruch 1, bei dem die erste reaktive Gruppe eine elektrophile Gruppe und die zweite reaktive Gruppe eine Abgangsgruppe ist.

26. Verfahren zur Herstellung eines Substrates mit daran gebundenem Biomolekül, Schritte umfassend, bei denen man
einen festen Träger bereitstellt, der aus polymeren Molekülen hergestellt wurde, von denen jedes eine erste reaktive Gruppe aufweist, wobei die erste reaktive Gruppe ohne Änderungen der Oberfläche des festen Trägers vorliegt; und eine Vielzahl von Biomolekülen, von denen jedes eine zweite reaktive Gruppe aufweist; wobei eine der beiden reaktiven Gruppen einen Substituent und die andere eine Abgangsgruppe darstellt; und
die Biomoleküle mittels einer chemischen Bindungsreaktion zwischen den zwei reaktiven Gruppen an einen festen Träger bindet, um ein Substrat mit daran gebundenem Biomolekül zu bilden.

27. Verfahren gemäß Anspruch 26, bei dem die erste reaktive Gruppe eine Abgangsgruppe und die zweite reaktive Gruppe eine nukleophile Gruppe ist.

28. Verfahren gemäß Anspruch 27, bei dem der feste Träger ein Polyvinylchlorid und die erste reaktive Gruppe ein Chlorid ist.

29. Verfahren gemäß Anspruch 27, bei dem die zweite reaktive Gruppe eine Phosphorthioat- oder eine Amino-Gruppe ist.

30. Verfahren gemäß Anspruch 29, bei dem der feste Träger ein Polyvinylchlorid und die erste reaktive Gruppe ein Chlorid ist.

31. Verfahren gemäß Anspruch 27, bei dem das Biomolekül ein Nukleotid oder ein Oligonukleotid ist.

32. Verfahren gemäß Anspruch 27, bei dem das Biomolekül eine Aminosäure oder ein Peptid ist.

33. Verfahren gemäß Anspruch 26, bei dem die erste reaktive Gruppe eine Abgangsgruppe und die zweite reaktive Gruppe eine elektrophile Gruppe ist.

34. Verfahren gemäß Anspruch 26, bei dem die erste reaktive Gruppe eine nukleophile Gruppe und die zweite reaktive Gruppe eine Abgangsgruppe ist.

35. Verfahren gemäß Anspruch 34, bei dem das Biomolekül ein Nukleotid oder ein Oligonukleotid ist.

36. Verfahren gemäß Anspruch 34, bei dem das Biomolekül eine Aminosäure oder ein Peptid ist.

37. Verfahren gemäß Anspruch 26, bei dem die erste reaktive Gruppe eine elektrophile Gruppe und die zweite reaktive Gruppe eine Abgangsgruppe ist.
